# EUROPEAN PATENT SPECIFICATION

(11) **EP 1 715 888 B1**
(45) Date of publication and mention of the grant of the patent: **01.08.2007**
(21) Application number: 05713491.8
(22) Date of filing: 11.02.2005
(51) Int. Cl.: A61K 38/48

(54) **TREATMENT OF ABNORMALITIES OF THE FIRST METATARSOPHALANGEAL JOINT OF THE FOOT**
BEHANDLUNG VON ABWEICHUNGEN DER ERSTEN METATARSOPHALANGEALEN VERBINDUNG DES FUSSES
TRAITEMENT DES ANOMALIES DE LA PREMIERE ARTICULATION METATARSOPHALANGIENNE DU PIED

(30) Priority: 12.02.2004 US 544017 P
(43) Date of publication of application: 02.11.2006
(73) Proprietor: Radovic, Philip, San Clemente, CA 92673 (US)
(72) Inventor: Radovic, Philip, San Clemente, CA 92673 (US)
(74) Representative: Carpintero Lopez, Francisco
(86) International application number: PCT/US2005/004599
(87) International publication number: WO 2005/079828

(56) References cited:
- WO-A-95/17904
- US-A1- 2003 224 020
- SHERMAN AL, WILLICK SP, CARDENAS DD: "Management of Focal Dystonia of the Extensor Hallucis Longus Muscle with Botulinum toxin injection: A case Report" ARCH PHYS MED REHABIL, no. 79, 1998, pages 1303-1305, XP002349918
- YELNIK AP, COLLE FM, BONAN IV, LAMOTTE DR: "Disabling overactivity of the extensor hallucis longus after stroke: clinical expression and efficacy of botulinum toxin type A" ARCH PHYS MED REHABIL, no. 84, 2001, - 2003 pages 147-149, XP002349919
- YELNIK A P, BONAN I V: "Past Stroke Hemiplegia: lower limb benefit from botulinum toxin (Review)" ANNALES DE R¹ADAPTATION ET DE M¹DECINE PHYSIQUE, vol. 46, 2003, pages 281-285, XP002349920
- GILADI N, MEER J, HONIGMAN S: "The use of botulinum toxin to treat "striatal" toes" J NEUROL NEUROSURG PSYCHIATRY, vol. 57, 1994, XP009055560
- FERRARI J: "Bunions" CLIN EVID., vol. 10, 2003, pages 1259-1270, XP001207400

## Description

This application claims priority to U.S. Provisional Application serial No. 60/544,017, which was filed on February 12, 2004.

### Field of the Invention

The present invention relates to methods of treating abnormalities of the first metatarsophalangeal joint of the foot.

### Background of the Invention

Abnormalities of the first metatarsophalangeal joint encompass a variety of disorders, including hallux abductovalgus (commonly known as "bunions"), hallux varus, hallux limitus, hallux rigidus, and other disorders.

Hallux abductovalgus ("hallux abductovalgus") is one of the most frequently seen abnormalities of the first metatarsophalangeal joint. In a patient with hallux abductovalgus, the proximal phalanx of the hallux (the great toe) points toward the second toe. This results in a lateral deviation of the great toe (tilting of the great toe away from the mid-line of the body) and a widening of the angle between the first and second metatarsals.

One of the greatest deforming forces in the development of hallux abductovalgus is the adductor hallucis muscle. This muscle has two muscle bellies, a transverse and an oblique. In a patient with hallux abductovalgus, the adductor hallucis muscle gains mechanical advantage, pulling the hallux laterally and forcing the metatarsal head medially.

The severity of hallux abductovalgus deformities has traditionally been quantified based on a variety of measurements from radiographs. One common measurement is the intermetatarsal angle between the line of the first and second metatarsal. Normally, this angle can average from about 6 to about 8 degrees. In a patient with hallux abductovalgus, the intermetatarsal angle is increased, with severe abnormalities measuring greater than 30 degrees. Another common measurement is the hallux abductus angle, which is the angle between the longitudinal axes of the first metatarsal and the great toe. Normally, this angle can average from about 10 to about 15 degrees. In hallux abductovalgus, the hallux abductus angle is increased, with extreme cases measuring greater than 70 degrees. A third common measurement is the tibial sesamoid position. With hallux abductovalgus, the first metatarsal deviates medially off of the sesamoids, causing apparent lateral dislocation. The position of the tibial sesamoid in relation to a line drawn through the mid-longitudinal axis of the first metatarsal determines the tibial sesamoid position.

Hallux varus is an abnormality of the first metatarsophalangeal joint in which the proximal phalanx of the hallux (great toe) points away from the second toe. This results in a medial deviation of the great toe (tilting of the great toe toward the mid-line of the body). A common deforming force in the development of hallux varus is the abductor hallucis muscle. In a patient with hallux varus, the abductor hallucis muscle can gain mechanical advantage, pulling the hallux medially and forcing the metatarsal head laterally.

Hallux limitus is an abnormality of the first metatarsophalangeal joint that results in a restricted range of motion in the first metatarsophalangeal joint. Normally, the range of motion in the first metatarsophalangeal joint can average from about 55 to about 75 degrees. In a patient with hallux limitus, this range of motion is decreased. When the range of motion becomes less than about 5 degrees, this condition is commonly referred to as hallux rigidus (stiff great toe).

Hallux limitus can be functional or structural. Functional hallux limitus exhibits a restricted range of motion in the first metatarsophalangeal joint only during weightbearing. Structural hallux limitus, on the other hand, exhibits a restricted range of motion in the first metatarsophalangeal joint both during weightbearing and non-weightbearing.

### Summary of the Invention

According to the present invention, a variety of abnormalities of the first metatarsophalangeal joint can be treated using neuromuscular toxins. For example, in one embodiment, hallux abductovalgus can be treated by administering to the patient an amount of toxin sufficient to alleviate a symptom of hallux abductovalgus. In preferred aspects, the toxin can be administered by intramuscular injection, preferably into the adductor hallucis muscle. Most preferably, toxin can be injected into both of the two muscle bellies of the adductor hallucis: the transverse and the oblique. In addition, toxin can be administered to the extensor digitorum brevis muscle, which is involved in extension of the great toe.

In another embodiment, hallux varus can be treated by administering an amount of toxin sufficient to alleviate a symptom of hallux varus. In preferred aspects, the toxin can be administered by intramuscular injection, preferably into the abductor hallucis muscle.

In another embodiment, hallux limitus (or in severe cases, hallux rigidus) can be treated by administering an amount of toxin sufficient to increase the range of motion in the first metatarsophalangeal joint. In preferred aspects, the toxin can be administered by intramuscular injection, preferably into the flexor hallucis brevis muscle. Most preferably, toxin can be injected into both the medial and lateral muscle bellies of the flexor hallucis brevis muscle.

Other abnormalities of the first metatarsophalangeal joint which can be treated in accordance with the present invention can include trauma, sesamoid disorders, and other disorders of the first metatarsophalangeal joint.

The toxin can be any neuromuscular toxin capable of interfering with the connection between muscle and nerve. In preferred aspects, the neuromuscular toxin is an inhibitor of acetylcholine release. For example, a clostridial toxin, such as botulinum toxin, preferably can be used. Currently, there are seven known serotypes of botulinum toxin, designated as types A through G. The most currently preferred neuromuscular toxin currently is botulinum toxin type A.

As described above, the preferred technique for administering the toxin is by intramuscular injection. For example, in preferred aspects of this embodiment, a needle can be inserted into the target muscle and the toxin injected into the muscle, repeating as necessary to deliver the desired amount of toxin to the muscle. In further preferred aspects of this embodiment, electrical stimulation can be used to determine the optimal sites for injection. Other methods of administering the toxin can also be used.

In another embodiment, the method optionally further comprises stimulating the muscle opposed to the muscle to which the toxin has been administered.

In another embodiment, the method optionally further comprises immobilizing the foot to maintain position after the toxin has been administered. It is further contemplated that post procedural immobilization can be used in conjunction with electrical stimulation of the opposing muscle.

In another embodiment, the toxin can be administered to the patient while undergoing surgery on the foot.

### Detailed Description of the Invention

The present invention relates to methods of treating abnormalities of the first metatarsophalangeal joint through the use of neuromuscular toxins. Practice of the invention involves administering to the patient an amount of toxin sufficient to alleviate a symptom of the joint abnormality. In preferred aspects, the toxin can be administered by intramuscular injection. Injection of small doses of neuromuscular toxin into the muscle induces an effect similar to denervation, resulting in dose-dependent loss of muscle tone and subsequent muscle atrophy.

The toxin can be any neuromuscular toxin capable of interfering with the connection between muscle and nerve. In preferred aspects, the toxin is an inhibitor of acetylcholine release, such as botulinum toxin or a protein that mimics its acetylcholine release inhibiting effect. Currently, there are seven known serotypes of botulinum toxin, designated as types A through G. Other potentially useful toxins include, but are not limited to, tetanus toxins, tetrodotoxin, difficile toxins, butyricum toxins, and various animal venoms. Staphylococcal alpha-toxin can also be used, as it has also been shown to induce reversible, flaccid paralysis of skeletal muscle. (Harshman, et al., Infect. Immun., 62:421-425, 1994).

Recombinant, synthetic, and derivative neuromuscular toxins are also contemplated by the invention. For example, proteins produced using recombinant DNA technology which mimic the effects of these natural toxins can be used. Suitable toxins can also include proteins synthetically produced using in vitro protein synthesizing techniques well known in the art. Synthetically produced neurotoxins are also intended to include substances which have been rendered neurotoxic by a variety of manipulations, such as enzymatic or chemical processing and conjugation or derivatization with moieties which themselves are neurotoxic. Accordingly, toxins for use in connection with the present invention include derivatives of naturally occurring toxins and other known toxins. "Derivative" means a chemical entity which is slightly different from a parent chemical entity but which still has a biological effect similar, or substantially similar, to the biological effect of the chemical entity. For example, suitable toxin derivatives can include neurotoxin components that have modified amino acid side chains, as is well known in the art.

The invention also contemplates that derivatives in the form of fragments, subunits, and chimeras of neuromuscular toxins can be used. Botulinum toxin, for example, is composed of a heavy chain and a light chain, joined together by two disulfide bonds. Through disruption of the disulfide bond, the subunits can be separated and combined with other moieties, such as stabilizers or toxicity enhancers. If re-associated with other subunits or toxic substances, a biologically active chimera suitable for use in the present invention can be produced. Toxin fragments, e.g., a portion(s) of neurotoxin that retains neurotoxic and/or biological activity, can also be used.

The invention also contemplates that neurotoxic substances that share amino acid sequence homologies and/or identities with currently known neuromuscular toxins can be used. In addition, mixtures of toxins can also be used, preferably where such mixtures have been selected to cause longer-lasting action than with a single toxin.

The currently preferred toxin is a botulinum toxin, most preferably botulinum toxin type A. Commercially available from Allergan (BOTOX) and Ipsen (DYSPORT), botulinum toxin type A is an artificially produced neuromuscular paralyzing agent that is currently licensed by the FDA for cervical dystonia, blepharospasm, strabismus, and wrinkles. When injected into muscle, the botulinum toxin binds to the nerve ending and blocks the nerve from releasing acetylcholine. As a result, the muscle cannot contract and effectively relaxes. Botulinum toxin type B is commercially available under the trademark MYOBLOC and has also been shown to be clinically safe and effective in treating a number of neuromuscular conditions. Use of botulinum toxin type F is also being investigated for commercialization.

The degree of muscle relaxation can be regulated by variation of dosage, variation in the method or site of administration, and frequency of administration.

The dose of toxin administered to the patient will depend upon the severity of the condition (*e.g.* the size of the area requiring treatment, the age and size of the patient and the potency of the toxin). One unit (U) of toxin is defined as the LD.sub.50 upon intraperitoneal injection into female Swiss Webster mice weighing 18 to 20 grams each. Typically, the dose administered to the patient may be from about 1 to about 1000 units. In one embodiment, the currently preferred dosage for botulinum toxin type A is from about 50 units to about 300 units. Although such a maximum far exceeds the dosage employed in the treatment of blepharospasms and dystonias (10-150 U), it is well below the lethal dose for humans (estimated to be about 3000 U). Most preferably, the range of dosage of botulinum toxin type A is from about 75 units to about 100 units. Those of ordinary skill in the art will know of or can readily determine without undue experimentation suitable dosages for other neuromuscular toxins.

Because the effects of neuromuscular toxins can be delayed, it is further contemplated that post procedural monitoring of the patient can be used to determine if further administration of the toxin is needed. For example, in the case of hallux abductovalgus, comparison of pre and post procedural measurements of the intermetatarsal angle, the hallux abductus angle, and/or the tibial sesamoid position can be used to determine whether further treatment is required. In a currently preferred aspect of this embodiment, such post procedural monitoring of the patient can be performed at about 3 to 6 weeks following the initial procedure. If such monitoring reveals that further treatment is required, toxin can be readministered as needed.

The effects of botulinum toxin A generally last for about 3 to about 6 months, depending on the patient. If symptoms recur, toxin can be readministered as needed. Frequency of administration for other neuromuscular toxins can be determined using routine experimentation by those skilled in the art.

As described above, the preferred method of administering the selected toxin is by injection into the target muscle. Intramuscular injection can be accomplished using any suitable injection device. For example, a 27-gauge needle in a 3-cc tuberculin syringe can be used to deliver the toxin directly into the muscle. Needle-less injection systems can also be used to inject the toxin into the target muscle.

Alternatively, those of ordinary skill in the art will be able to determine other suitable techniques for administering the toxin. For example, transdermal delivery systems can be used to administer the toxin as needed. In addition, the toxin can be administered during surgery on the foot, in which case any suitable technique for delivering the toxin to the target area during surgery can be used.

If administered by intramuscular injection, those of ordinary skill in the art will be able to determine suitable techniques for injecting the toxin. In currently preferred aspects of this embodiment, electrical stimulation can be used to determine the optimal sites for injection. For example, an injectable needle attached to an electrode can be inserted through the skin and into the target muscle. This needle electrode can then be attached to the stimulator probe of a standard electrical stimulation unit. As the needle is advanced into the muscle, electrical stimulation is delivered to elicit a motor response. As the stimulated muscle responds by contracting, visual identification can be used to confirm that the needle is properly located in the target muscle.

For example, if toxin is to be administered by intramuscular injection to treat hallux varus, the abductor hallucis muscle preferably can be palpated at the medial aspect of the foot and the needle electrode placed from the medial skin directed into the mid belly of the abductor hallucis muscle. Once a motor response is elicited (hallux adduction), the toxin can be injected.

Those of ordinary skill in the art will know of, or can readily ascertain, other suitable techniques for injecting the toxin, if intramuscular injection is to be used. For example, depending on the muscle to be injected, electromyography can be used, alone or in combination with electrical stimulation, to determine the optimal sites for injection. Alternatively, those of ordinary skill in the art may be able to determine the optimal sites of injection anatomically. In addition, those of ordinary skill will appreciate that in some cases there may be reasons to administer the toxin to suboptimal sites. In each case, this process can be repeated as necessary to deliver sufficient toxin to the target area.

In another embodiment, the method optionally further comprises stimulating the muscle opposed to the muscle to which the toxin is administered. In most cases, such further stimulation is unnecessary. When used, stimulation of the opposing muscle can be achieved by using a standard electric muscle stimulator to deliver electronic impulses to the opposing muscle. For example, in the case of hallux abductovalgus, stimulation can be applied by placing electrode pads preferably over the motor points of the abductor hallucis and delivering low volt stimulation to cause a muscle contraction. Most preferably, stimulation of the opposing muscle can be performed by the patient as needed following administration of the neuromuscular toxin. For example, in currently preferred aspects of this embodiment, the patient can be instructed to stimulate the opposing muscle on a daily basis before coming in for follow-up.

In another embodiment, the method optionally further comprises immobilizing the foot to maintain position after the toxin has been administered. The use of immobilization to maintain position following corrective procedures for abnormalities of the first metatarsophalangeal joint is well known in the art. For example, a splint, surgical shoe, ridged sole shoe, casting, gauze, tape, or the like can be used to immobilize to foot following administration of the toxin. In a currently preferred aspect of this embodiment, a standard splint can be placed on the foot after the toxin has been administered. For example, in the case of hallux abductovalgus, the patient preferably can be instructed to place the foot in a bunion splint on a nightly basis before coming in for follow-up.

In another embodiment, immobilization can be used in conjunction with electrical stimulation of the opposing muscle. For example, in the case of hallux abductovalgus, the patient preferably can be instructed to stimulate the abductor hallucis muscle while placing the foot in a bunion splint on a daily basis before coming in for follow-up.

In another embodiment, the toxin can be administered to the patient during surgery on the patient's foot. In preferred aspects, toxin can be administered to the target muscle during surgery for the joint abnormality, preferably after the primary surgical treatment has been carried out. For example, in the case of hallux abductovalgus, the toxin preferably can be administered by intramuscular injection into the adductor hallucis muscle during surgery on the first metatarsophalangeal joint, preferably after the primary surgical treatment has been carried out.

### Examples

The invention will now be illustrated by reference to the following nonlimiting examples.

In each example, appropriate areas were injected with a sterile solution containing Botulinum toxin (e.g. 100 units BOTOX solubilized in 0.9% sterile saline without preservative). Determination of the site to inject was performed using a DIGISTIM III peripheral nerve/muscle stimulator and an INOJECT needle electrode, with placement of the lead (gel electrode) in the patient's thigh.

### Example 1

A female patient suffering from hallux abductovalgus was treated with 100 units of botulinum toxin type A by direct injection of the toxin into the adductor hallucis muscle. Determination of the injection sites was performed by placing the INOJECT needle from the dorsal mid first interspace of the foot proximal to the first and second metatarsophalangeal joint and delivering a 2 Hz pulse while advancing the needle in the direction of the transverse adductor hallucis muscle belly. Once a motor response was elicited (pulsating abduction of the hallux), 25 units of the toxin were injected into the transverse belly of the adductor hallucis muscle. The needle was then partially retracted and redirected upward toward the oblique arm of the adductor hallucis muscle and advanced plantarly until a motor response was elicited (adduction of the hallux). At this point, 75 units of the toxin were injected into the oblique adductor hallucis muscle. Within 1 week, the symptoms of hallux abductovalgus were markedly reduced.

The patient was followed for 41 days following injection, with pre and post procedure measurements of radiographs obtained to monitor the patient's progress. Before the procedure, the patient exhibited an intermetatarsal angle of 14 degrees, a tibial sesamoid position of 4, and a hallux abductus angle of 20 degrees. Twenty four days following injection, the patient's intermetarsal angle was reduced to 10 degrees, her tibial sesamoid position was 3, and her hallux abductus angle was 10 degrees. Forty one days following injection, the patient's intermetarsal angle was further reduced to 9 degrees, her tibial sesamoid position was 2, and her hallux abductus angle was 7 degrees. Through day 41, the patient has reported none of her previous symptoms associated with hallux abductovalgus.

### Example 2

A female patient suffering from hallux limitus was treated with 100 units of botulinum toxin type A by direct injection of the toxin into the flexor hallucis brevis muscle. Determination of the injection sites was performed by placing the INOJECT needle through the skin from the dorsum of the foot at the mid first interspace proximally and applying stimulation while advancing the needle in a plantar medial direction to the lateral belly of the flexor hallucis brevis. Once location was confirmed with a motor response of plantar flexion of the first metatarsophalangeal joint, 50 units of toxin were injected into the lateral belly of the flexor hallucis brevis muscle. The medial belly of the flexor hallucis brevis muscle was then approached with the needle through the medial aspect of the foot where the muscle can be palpated. The needle was advanced transversely from about the level of the first metatarsal until a motor response of plantar flexion of the first metatarsophalangeal joint was elicited. At this point, 50 units of toxin were injected into the medial belly of the flexor hallucis brevis muscle.

Within 3 days, the symptoms of hallux limitus were markedly reduced. Before treatment, the patient exhibited a range of motion at the first metatarsophalangeal joint of 30 degrees with pain upon range of motion. One week after injection, the patient exhibited a range of motion of 50 degrees at the first metatarsophalangeal joint with no pain at the end range of motion. At week 6, the patient exhibited a range of motion of 55 degrees at the first metatarsophalangeal joint with no pain upon range or end range of motion.

While particular forms of the invention have been described, it will be apparent that the invention can be embodied in other specific forms without departing from the spirit and scope thereof.

## Claims

1. Use of a neuromuscular toxin in the preparation of a medicament for the treatment of hallux abductovalgus, hallux varus, hallux limitus, or hallux rigidus.

2. The use of claim 1, wherein the abnormality is hallux abductovalgus.

3. The use of claim 1, wherein the neuromuscular toxin is a botulinum toxin.

4. The use of claim 3, wherein the neuromuscular toxin is botulinum toxin type A.

5. The use of claim 4, wherein the unit dose of the medicament comprises between about 50 units and about 300 units of botulinum toxin type A.

6. The use of claim 3 wherein the neuromuscular toxin is botulinum toxin type B.

7. The use of claim 1, wherein the neuromuscular toxin is a mixture of toxins.

8. The use of claim 1, wherein the abnormality is associated with mechanical advantage of the adductor hallucis muscle.

9. The use of claim 1, wherein the abnormality is associated with mechanical advantage of the abductor hallucis muscle.

10. The use of claim 1, wherein the abnormality is associated with mechanical advantage of the flexor hallucis brevis muscle.

## Patentansprüche

1. Anwendung eines neuromuskulären Toxins zur Herstellung eines Medikaments zur Behandlung eines Hallux (abducto)valgus, Hallux varus, Hallux limitus oder Hallux rigidus.

2. Anwendung gemäß Anspruch 1, wobei die Fehlbildung bzw. Anomalie ein Hallux abductovalgus ist.

3. Anwendung gemäß Anspruch 1, wobei das neuromuskuläre Toxin ein Botulinumtoxin ist.

4. Anwendung gemäß Anspruch 4, wobei das neuromuskuläre Toxin ein Botulinumtoxin Typ A ist.

5. Anwendung gemäß Anspruch 4, wobei die Einheitsdosis des Medikaments zwischen 50 Einheiten und ungefähr 300 Einheiten des Botulinumtoxins Typ A beinhaltet.

6. Anwendung gemäß Anspruch 3, wobei das neuromuskuläre Toxin ein Botulinumtoxin Typ B ist.

7. Anwendung gemäß Anspruch 3, wobei das neuromuskuläre Toxin eine Mischung von Toxinen ist.

8. Anwendung gemäß Anspruch 1, wobei die Fehlbildung bzw. Anomalie assoziiert ist mit dem mechanischen Nutzen bzw. Vorteil des M. (Musculus) adductor hallucis.

9. Anwendung gemäß Anspruch 1, wobei die Anomalie assoziiert ist mit dem mechanischen Vorteil des M. abductor hallucis.

10. Anwendung gemäß Anspruch 1, wobei die Anomalie assoziiert ist mit dem mechanischen Vorteil des M. flexor hallucis brevis.

## Revendications

1. Utilisation d'une toxine neuromusculaire dans la préparation d'un médicament pour le traitement du hallux abductovalgus, du hallux varus, du hallux limitus, ou du hallux rigidus.

2. Utilisation selon la revendication 1, dans laquelle l'anomalie est le hallux abductovalgus.

3. Utilisation selon la revendication 1, dans laquelle la toxine neuromusculaire est une toxine botulique.

4. Utilisation selon la revendication 3, dans laquelle la toxine neuromusculaire est la toxine botulique de type A.

5. Utilisation selon la revendication 4, dans laquelle la dose unitaire du médicament comprend entre environ 50 unités et environ 300 unités de toxine botulique de type A.

6. Utilisation selon la revendication 3, dans laquelle la toxine neuromusculaire est la toxine botulique de type B.

7. Utilisation selon la revendication 1, dans laquelle la toxine neuromusculaire est un mélange de toxines.

8. Utilisation selon la revendication 1, dans laquelle l'anomalie est associée à un avantage mécanique du muscle adducteur du gros orteil.

9. Utilisation selon la revendication 1, dans laquelle l'anomalie est associée à un avantage mécanique du muscle adducteur du gros orteil.

10. Utilisation selon la revendication 1, dans laquelle l'anomalie est associée à un avantage mécanique du muscle court fléchisseur du gros orteil.
